(19) Europäisches Patentamt
European Patent Office
Office européen des brevets

(11) Publication number: 0 188 093
A2

(12) EUROPEAN PATENT APPLICATION

(21) Application number: 85309043.9

(22) Date of filing: 12.12.85

(51) Int. Cl.[4]: G 01 N 33/543

(30) Priority: 12.12.84 US 680730

(43) Date of publication of application:
23.07.86 Bulletin 86/30

(84) Designated Contracting States:
AT BE CH DE FR GB IT LI LU NL SE

(71) Applicant: IMMUNOMEDICS, INC.
100 Bergen Street, Building 5
Newark New Jersey 07103(US)

(72) Inventor: Primus, Frederick James
R.D. 1, Box 6 Pittstown
New Jersey 08867(US)

(74) Representative: Sheard, Andrew Gregory et al,
Kilburn & Strode 30, John Street
London WC1N 2DD(GB)

# (54) Sandwich immunoassay.

(57) An immunoassay system for determining the amount of a polyvalent ligand in a liquid test sample is based on:

(a) an insoluble phase to which is bound a separation specific binding substance which does not bind the ligand;

(b) a capture specific binding substance which specifically binds the ligand, and which is itself specifically bound by the separation specific binding substance; and

(c) a probe specific binding substance which specifically binds the ligand at a site which is accessible when the ligand is bound to the capture specific binding substance, the probe specific binding substance being not substantially bound by the separation specific binding substance, and being detectable by at least one detection procedure.



Croydon Printing Company Ltd.

SANDWICH IMMUNOASSAY

The present invention relates to a method and kit useful for detemining the amount of a ligand in a liquid test sample.

Heterogeneous immunoassays for detection of a ligand in a liquid test sample have used a variety of methods to separate antigen complexed with antibody from free antigen. Some of these methods exploit physicochemical properties of the antigen, such as chemical solubility, molecular size, adsorption properties and electrophoretic mobility, whereas others employ immunological techniques in which an antibody which specifically binds the ligand-specific antibody (separation antibody) is used. Several known methods are base don the attachment of the anti-antigen antibody or the antigen itself to a solid phase such as polystyrene, the separation of components being effected by washing steps.

Examples of the variety of such immunoassays are found in, e.g., US patents 4,228,237, 3,879,262, 4,021,534, 4,273,756, 4,098,876, 4,230,683, 4,048,298, 4,243,749, 4,343,896, 4,315,907, 4,332,783, 4,320,109, 4,298,685, 4,185,084, 4,312,944, to cite just a few typical examples. The disclosures of the foregoing patents are hereby incorporated herein by reference in their entireties. Although all of these methods are useful, each suffers from certain limitations, such as the need for purified antigen, the need to attach the antigen to a solid phase, the need for the antigen to have certain physicochemical properties, the use of multiple washing and incubating steps, and the like.

A need therefore continues to exist for a method and kit for immunoassays which avoids these disadvantages.

One object of the present invention is to provide an immunoassay method suitable for determining the amount of a polyvalent ligand in a liquid test sample, wherein the method can use a solid support that is adapted for use with any ligand, i.e., a "universal separation system".

Another object of the present invention is to provide an immunoassay method that avoids the need for multiple washing and incubation steps.

A further object of the present invention is to provide an immunoassay method that can be used to effect simultaneous multiple antigen assays of a single liquid test sample.

Yet another object of the invention is to provide a test kit for use in effecting the method of the invention.
Other objects and advantages of the invention will become more readily apparent to those skilled in the art upon further study of the specification and appended claims.

According to a first aspect of the invention, there is provided an immunoassay method for determining the amount of a polyvalent ligand in a liquid test sample, the ligand being a binding substance such as an antibody or a substance capable of specific binding to a binding substance such as an antibody, the method comprising the steps of:

(a) providing an insoluble phase to which is bound a separation specific binding substance which does not bind the ligand;

(b) incubating the insoluble phase with

(1) the liquid test sample;

(2) a capture specific binding substance which specifically binds the ligand, and which is itself specifically bound by the separation specific binding substance; and

(3) a probe specific binding substance which specifically binds the ligand at a site which is accessible when the ligand is bound to the capture specific binding substance, the probe specific binding substance being not substantially bound by the separation specific binding substance, and being detectable by at least one detection procedure;

(c) separating the insoluble phase after the incubation, from resultant liquid phase containing unbound reagents and components of the test sample; and

(d) determining, by means of the detection procedure, the amount of the detectable probe which is bound to the separated insoluble phase, or the amount of the detectable probe remaining is the resultant liquid phase, the determination being related to the amount of the ligand in the test sample.

The invention further provides an immunoassay kit for use in carrying out the foregoing method.

According to a second aspect of the invention, there is provided an immunoassay kit, suitable for determining the amount of a polyvalent ligand in a liquid test sample, the kit comprising:

(a) an insoluble phase to which is bound a separation specific binding substance which does not bind the ligand;

(b) a capture specific binding substance which specifically binds the ligand, and which is itself capable of being specifically bound by the separation specific binding substance; and

(c) a probe specific binding substance which specifically binds the ligand at a site which is accessible when the ligand is bound to the capture specific binding substance, the probe specific binding substance being not substantially bound by the separation specific binding substance, and being detectable by at least one detection procedure.

A major advantage of the method and kit of the present invention is that the specific binding substance bound to the solid phase does not bind the ligand, but instead binds the capture specific binding substance. This permits considerable flexibility to be achieved which is not possible in other sandwich assay systems.

The ligand may be an antibody, and the capture specific binding substance may comprise a specific binding complement to the antibody.

Where the ligand is a substance capable of binding specifically to an antibody but is not itself an antibody, i.e., the substance is a specific binding complement to the antibody, the capture specific binding substance may be an antibody which specifically binds the ligand. The separation specific binding substance bound to the solid phase may then be either an antibody that specifically binds the capture antibody, e.g., a subtype-specific or species-specific antibody, or an antibody that specifically binds a hapten that is conjugated to the capture antibody. Examples of the latter configuration can include a capture antibody that is conjugated to a hapten such as, e.g., a steroid, a fluorochrome, p-azobenzene arsenate, p-azotrimethyl-anilinium, benzoyl-L-glutamic acid, 2,4,6-trinitrophenyl and the like.

It will be understood that the term "antibody" as used herein means a polyclonal or monoclonal whole immunoglobulin, e.g., IgG, IgM, IgA, IgE and the like, or an immunoglobulin fragment, e.g., F(ab)2, F(ab')2, Fab, Fab' and the like, or a mixture thereof, and includes synthetic antibody. Antibodies and antibody fragments which specifically bind a wide variety of ligands are known, and many of these are disclosed in the patents whose disclosures are incorporated herein by reference, such species being illustrative of representative genera. Many antibodies and antibody fragments which specifically bind tumour-associated markers are disclosed in, e.g., U.S. Patents 4,348,376, 4,361,544, 4,331,647, 4,468,457, 4,444,744, 4,460,559 and 4,460,561, the disclosures of which are incorporated herein by reference, such antibodies and antibody fragments being illustrative of representative genera.

Other antibodies useful in the method and immunoassay kit of the invention are conventional and known to the art of can be produced by conventional immunological techniques, the particular method of preparation being within the ordinary skill of the art worker in light of the particular ligand to be assayed. For example, antibodies to enzymes, e.g., kinases such as creatinine kinase, dehydrogenases such as liver dehydrogenases and the like, hormones, e.g., luteinizing hormone, follicle stimulating hormone, steroids such as progesterone, testosterone and the like, virus or viral fragments, fungi, bacteria, protozoa or other disease causing or disease related microorganisms, can all be prepared and/or are known, and can be used as capture and/or probe antibodies in the method and kit of the present invention.

A particularly attractive type of hapten is a moiety containing a carborane ring system. Carboranes are not known to occur in natural biological samples such as serum, urine and the like, so that their use as haptens avoids the possibility of cross-reactivity with components of the test sample. Antibodies to carboranes can be developed by conventioal techniques. Another attractive type of hapten is biotin, which can be conjugated to the capture specific binding substance; this conjugate would be used with avidin as the separation specific binding substance conjugated to the solid phase.

Use of any of the foregoing types of haptens permits the solid phase to function as a "universal" separation medium regardless of the nature of the ligand or the capture specific binding substance. All that is required is that the capture species contain structural features that can be recognized by the separation specific binding substance, either by virtue of characteristic regions on an isotype, for example, or by virtue of the features of the hapten conjugated to the capture substance.

As an illustration, if the ligand to be determined is carcinoembryonic antigen (CEA) - an example of tumour-associated markers, which are suitable ligands - the capture specific binding substance is advantageously an antibody which specifically binds CEA, e.g., a polyclonal antiserum with high specific activity towards CEA, or a monoclonal antibody which specifically binds CEA. The probe would advantageously be another antiserum or monoclonal which specifically binds CEA, and which is conjugated to, e.g., a radioisotope, a fluorescent marker, an enzyme or the like detectable moiety. (Such labels may also be used with other embodiments.) For the sake of this illustration, the probe will be a monoclonal anti-CEA antibody to which is conjugated peroxidase. Again, for ease of illustration, the capture substance may be either the same or a different anti-CEA monoclonal antibody to which is conjugated a diazophenylcarborane. The solid phase is, in this illustration, a polypropylene test tube to which is conjugated monoclonal antibodies to the carborane ring of the hapten bound to the capture antibody.

The assay would proceed by adding an aliquot of the liquid test sample suspected to contain CEA, e.g., serum from a patient who has had surgery for removal of a colorectal tumour. To the sample would be added a solution of the phosphate-buffered saline, and the resultant solution would be incubated for a period of time sufficient for binding the capture antibody to the solid phase, binding the ligand to the bound ligand. It will be understood that the reagents can be added sequentially as well as all together, and the order of their addition is not critical, although it may be advantageous to add the capture antibody first and the probe antibody afterwards rather than add these reagents in the inverse order.

Once the incubation is complete, hydrogen peroxide is added, together with o-phenylenediamine, and the activity of the peroxidase is determined spectrophotometrically, by conventional means. The activity can be correlated to the concentration of the enzyme, which in turn is related to the amount of CEA in the test sample by conventional means.

A simple variant of the above illustrated procedure could use I-125 as the label for the probe antibody.

In this case, the tube would be drained after incubation and counted for bound radiolabel, which in turn would be related to the amount of CEA in the test sample.

Another simple variant would use biotin as the hapten conjugated to the capture antibody, and either an enzymatic or radioisotopic probe label. The solid phase would have avidin conjugated thereto, e.g., by conventional means, and any ligand bound by the capture antibody would be linked through the biotin-avidin couple to the solid phase and would be rendered detectable by the labelled probe bound to the ligand at a separately accessible site.

Other tumour-associated markers which may be used as ligands include colon-specific antigen-p, human chorionic gonadotrophin or its beta-subunit, alpha-fetoprotein, meconium antigen and prostatic acid phosphatase.

In order to use the method of this invention for the simultaneous determination of two or more ligands in the same test sample, the same hapten can be conjugated to a capture antibody for each ligand.

More generally, according to a third aspect of the invention, there is provided an immunoassay method for determing the amount of each of a plurality of polyvalent ligands in a liquid test sample, each of the ligands being a binding substance or a substance capable of specific binding to a binding substance, the method comprising the steps of;

(a) providing an insoluble phase to which is bound at least one separation specific binding substance which does not bind any of the ligands;

(b) incubating the insoluble phase with

(1) the liquid test sample;

(2) for each of the ligands, a capture specific binding substance which specifically binds that ligand, and which is itself specifically bound by the separation specific binding substance; and

(3) for each of the ligands, a probe specific binding substance which specifically binds that ligand at a site which is accessible when the ligand is bound to the capture specific binding substance, each of the probe specific binding substances being not substantially bound by the separation specific binding substance, and each being separately detectable by at least one detection procedure;

(c) separating the insoluble phase, after the incubation, from the resultant liquid phase containing unbound reagents and components of the test sample; and

(d) determining, by means of the detection procedure, the amount of each of the detectable probes which is bound to the separated insoluble phase, or the amount of the detectable probe remaining in the resultant liquid phase, the determination being related to the amount of the ligand in the test sample.

Preferred features of this aspect of the invention are, as far as possible, the same as for the first aspect.

If, for example, it is desired to determine the amounts of both CEA and colon-specific antigen-p (CSAp), monoclonal antibodies to CEA and CSAp are ech conjugated to, e.g., biotin, and the probe for CEA, another monoclonal which specifically binds CEA at a site which is accessible when CEA is bound to the capture antibody, is conjugated to, e.g., peroxidase, while the probe antibody for CSAp, another monoclonal that specifically binds CSAp at a site which is accessible when CSAp is bound to the capture antibody, is labelled with, e.g., glucose oxidase. The test sample is then incubated with the solid phase, to which is bound avidin, and with the capture and probe antibodies, after which sequential determinations are effected with, e.g., hydrogen peroxide and o-phenylenediamine for the peroxidase determination, and with, e.g., glucose, o-dianisidine and peroxidase for the glucose oxidase determination, and the reuslts correlated with standard curves.

Where the ligand is an antibody, it will often be convenient to use an anti-idiotype antibody as the capture or probe antibody, in conjunction with a probe or capture antibody that recognizes the isotype of the ligand antibody, each being conjugated with a function that can be bound by the separation specific binding substance on the solid phase or that can be detected, respectively. This avoids the need to use purified antigen as a component of the assay system. For example, if the ligand to be assayed is a human antibody to hepatitis-B surface antigen, the capture antibody could be a murine monoclonal anti-idiotype which specifically binds the hypervariable region of the ligand antibody and to which is conjugated I-125 in detectable quantities. The separation is effected by an anti-carborane antibody conjugated to the solid support, and the assay is effected by determining the amount of radioiodine bound to the solid support after incubation with the sample, in the presence of the capture and probe antibodies.

Immunoassay kits will normally contain a solid phase to which is bound the separation specific binding substance, e.g., polystyrene test tubes, microtiter plates and the like, coated with anti-hapten antibodies; lyophilized capture specific binding substances to which are conjugated haptens complementary to the separation specific binding substances, e.g., carborane-conjugated anti-ligand antibodies and the like; lyophilized probe specific binding substances labelled with detectable markers, e.g., enzyme-, radioisotope-, fluroescent marker-labelled anti-ligand antibodies and the like; ligand standards; and developers for the labels where necessary. Other conventional components can be included and these will be readily apparent to the skilled art worker.

Ligands may be infectious organisms, or parts of them, such as viruses, bacteria, fungi and protozoa.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following examples, all temperatures are set forth uncorrected in degrees Celsius; unless otherwise indicated, all parts and percentages are by weight.

## EXAMPLE 1

Immunoassay for CSAp

A) Murine monoclonal antibodies to CSAp are prepared according to the method of US Patent 4,468,457, the disclosure of which is incorporated herein in its entirety by reference. This method involves producing a tryptic peptide having the immunological characteristics associated with native CSAp, by trypsin digestion under controlled conditions using this tryptic peptide as an antigen, and generating murine monoclonal anti-CSAp IgG antibodies using conventional procedures.

B) The antibodies prepared above are treated with a solution of p-(1,2,-dicarbacloso-dodecaboranyl) benzenediazonium chloride, according to the method of Mizusawa et al., 1982, Proc. Nat. Acad. Sci., USA, 79: 3011-3014, to introduce an average of 7 carborane groups onto the antibody. This can be done without significant loss of immunoreactivity, and further improvement can be attained by effecting the introduction of the carborane functions in the presence of the tryptic peptide to protect the hypervariable region of the antibody, after which the antigen is removed by the use of a chaotropic agent.

Details of the method of Mizusawa et al are as follows: Antibody samples were dialysed overnight against normal saline before coupling. p-(1,2-Dicarba-closo -dodecaboranyl) benzenediazonium chloride solution was added to antibody samples of known concentrations contained in an ice bath, and the pH was adjusted with 0.05M NaOH. The solution was allowed to react overnight at 4°C. After dialysis of the conjugate, e.g., normal saline at 4°C, the carborane-conjugated antibody is purified by affinity chromatography on a Sepharose 4B immunoadsorbent to which is bound the CSAp tryptic peptide.

C) Monoclonal antibodies to the carborane moiety are prepared by conjugating the p-(1,2-dicarba-closo -dodecaboranyl) benzenediazonium chloride to bovine serum albumin, injecting the conjugate into Balb/c mice, together with Freund's complete adjuvant, injecting the mice with boosters of the conjugate, and sacrificing the mice after 8 weeks. The spleens of the mice are excised and splenocytes are isolated, fused with murine myeloma cells, by conventional procedures, and clones isolated which secrete antibodies which specifically bind carborane moieties, as determined by enzyme immunoassay using carborane-goat IgG conjugates attached to the wells of microtiter plates.

D) The anti-carborane antibodies prepared in part C are coated onto the inside of polystyrene test tubes according to the method disclosed in US Patent 4,185,084, the disclosure of which is incorporated herein by reference. By such a method, 1 ml of a buffered solution of antibody (20mcg/ml in glycine buffer, pH 8.2) was put into a polystyrene test tube and incubated for 30 minutes in a water bath at 56°C.

E) Another monoclonal anti-CSAp antibody, prepared according to the procedure of US 4,468,457, is conjugated to peroxidase, according to the procedure of Wilson and Nakane (1978), in "Immunofluorescence and Related Staining Techniques", Knapp et al., eds., pp. 215-224 (Elsevier/North Holland Biomedical Press, Amsterdam). This conjugate, prepared as follows, is used as the probe antibody for the assay.

Step 1 - dissolve 4mg horseradish peroxidase (HRPO) (RZ > = 3.0) in 1 ml distilled water.

Step 2 - add 200 microlitres freshly made 0.1M $NaIO_4$. Stir at room temperature 20 minutes.

Step 3 - dialyze the resulting HRPO-aldehyde overnight against 1mM sodium acetate buffer, pH 4.0. Also, dialyze 8mg IgG in about 1ml overnight against 0.01M sodium carbonate buffer, pH 9.5.

Step 4 - add 20 microlitres 0.02M sodium carbonate buffer, pH 9.5, to the HRPO-aldehyde (to raise pH 9-9.5) and immediately add the dialyzed 8mg IgG. Stir gently at room temperature 2 hours.
Step 5 - add 0.1ml of freshly made 4mg $NaBH_4$/ml $H_2O$. Leave at 4°C for 2 hours.

Step 6 - if conjugate mixture will not be chromatographed immediately dialyze against PBS (0.01M sodium phosphate buffered saline).

Step 7 - chromatograph on an 85 x 1.5cm or 35 x 2.5cm column of Sephadex G-100 or Sephacryl S-200 equilabrated in PBS. Read the absorbents off the fractions at 280 and 403nm.

F) A liquid serum sample from a patient suspected of having colorectal cancer is used as the test sample, after being freed of cellular matter by centrifugation. The sample is introduced into the polystyrene test tube prepared according to part D, and a solution of the capture and probe antibodies, prepared according to parts B and E, respectively, is introduced and incubated for 2 hours, at 37°C. The tube is washed with saline solution. A solution consisting of 0.08% o-phenylenediamine and 0.012% hydrogen peroxide in 0.1M phosphate-citrate buffer, pH 5.0, is then introduced into the tube, and allowed to incubate for 30 minutes, at 20°C. The tube is then introduced into a spectrophotometer, determined, and correlated with CSAp content of the sample by the use of a standard curve, determined with a series of dilutions of known concentrations of the CSAp tryptic peptide.

EXAMPLE 2

Simultaneous Immunoassay for CSAp and CEA using Enzyme-

labelled Probe Antibodies.

A) Murine monoclonal antibodies t CEA are prepared according to Primus et al. (1983), Cancer Res., 43:686-692. This method involves the preparation of monoclonal antibodies against CEA purified from liver metastases of colonic adenocarcinoma and specificity characterization through reactivity with the cross-reactive antigens, nonspecific cross-reacting antigen and meconium antigen. In greater detail, the procedure was as follows:

Preparation of NP-2 monoclonal antibody

Carcinoembryonic antigen (CEA), used for mouse immunization, was isolated from liver metastases of a colonic adenocarcinoma and radioiodinated by the chloramine-T method (Greenwood et al., Biochem J. (1963) 89 114-123) with $^{125}I$ (Amersham/Searle Corp., Arlington Heights, Ill., USA) to a specific activity of approximately 30 Ci/g. Alternatively, radiolabelled CEA from the Roche CEA assay kit (Nutley, NJ, USA), which was found to give results similar to those obtained with the CEA used for immunisation.

**Mouse Immunization.** Female BALB/c mice (Harlan-Sprague-Dawley, Indiapolis, Ind. USA). 3 to 4 months old, were given 3 i.p. injections of 20 mcg of CEA in incomplete Freund's adjuvant. The second and third injections were separated from the first by 2 and 8 weeks, respectively. Six months after the initial immunization, 2 mice demonstrating serum antibody against CEA were selected as spleen cell donors and received a final series of CEA injections, 50 mcg each in 0.9% NaC1 solution, accoridng to the immunization protocol of Stahli et al (J. Immunol. Methods (1981) 32, 297-304). At 4 days and 1 day prior to fusion, the CEA was injected i.p., while at 3 and 2 days before fusion it was equally divided for both i.p. and i.v. injections.

**Cell Fusion and Cloning.** Two separate fusions were performed with spleen cells obtained from 2 CEA-immunized mice according to the method of McKearn ("Fusion of cells on an adherent monolayer" in "Monoclonal Antibody Hybridomas: A New Dimension in Biological Analyses" (Kennett et al, Eds) pp 368-369, New York, Plenum Publishing Corp., 1980). For each fusion, 5 x $10^7$ Ficoll:Hypaque-separated spleen cells and 5 x $10^6$ P3-X63-Ag8.653 myeloma cells (Salk Institute, San Diego, Calif, USA) were mixed in a 60-mm culture dish and centrifuged at 250 x g for 5 min. Excess medium was removed and replced with 1.0 ml of 50% (v/v) polyethylene glycol 1500 (Fisher Scientific Co.) in Dulbecco's modified Eagle's medium (Grand Island Biological Co., Grand Island, NY, USA) at 37$^{o}$. After a 30-sec exposure to polyethylene glycol, the cells were washed twice and then incubated overnight in 5 ml of Dulbecco's modified Eagle's medium supplemented with 20% agamma horse serum (KC Biologicals, Inc., Lenexa, Kans, USA). 2mM L-glutamine, 1mM sodium pyruvate, 0.55 mM L-arginine, 0.27mM L-asparagine, and 14mcM folic acid. The cells were dispersed in 30 more ml of the latter medium further supplemented with 0.1mM hypoxanthine, 0.4mcM aminopterin, and 16mcM thymidine. The cell suspension was distributed into 96-well microwell plates (Costar, Cambridge, Mass, USA) 100 l/well. An additional 100mcl of medium minus the aminopterin were added to the wells 7 days later. After 2 to 4 weeks of culture, the medium from wells containing growing cell clones was assayed for CEA antibody by RIA. CEA antibody-positive wells were expanded into 24-well plates (Costar) containing $10^7$ irradiated (1400 R) Lewis rat thymocytes per well. Upon reaching confluence, all cultures remaining antibody positive were frozen, while 4 clones were twice recloned at limiting dilution. Hybridomas were recloned in 96-well plates containing $10^6$ irradiated Lewis rat thymocytes per well. The selected recloned hybridoma cell lines were expanded in culture flasks containing Dulbecco's modified Eagle's medium supplemented with 10% agamma horse serum, 2mM L-glutamine, and 1mM sodium pyruvate.

**Monoclonal Antibody Purification.** Monoclonal antibody was purified from culture media containing 10% agamma horse serum. Briefly, immunoglobulin was precipitated with 50% $(NH_4)_2SO_4$ at pH 7.0, resolubilized in

distilled $H_2O$, and reprecipitated with polyethylene glycol 7000 to 9000 at 13% (w/v) final concentration. The polyethylene glycol precipitate was solubilized in 0.02M phosphate (ph 5.6) and applied to a carboxymethylcellulose (Whatman, Inc., Clifton, NJ, USA) column equilibrated in the same buffer. Adsorbed immunoglobulin was eluted with 0.02M phosphate (pH 7.8):0.2M NaCl, dialyzed against 0.01M phosphate (pH 8.0), and applied to a DEAE-cellulose (Whatman) column equilibrated with the latter buffer. Adsorbed antibody was eluted with 0.025M phosphate (pH 8.0), equilibrated against PBS, and concentrated by ultrafiltration (Amicon, Danvers, Mass, UISA). The purified monoclonal antibodies were radioiodinated by the chloramine-T method to a specific acitivity of 5 to 10 mcCi/ug. In addition to mouse IgG, the purified antibody contained 10 to 40% horse immunoglobulin as determined by binding of the radioiodinated preparation to solid-phase goat anti-horse IgG. The percentage of monoclonal anti-CEA antibody in the preparation was determined by binding of the radioiodinated antibody to a CEA immunoadsorbent, as described previously (29).

Antibody isotype was determined by double immunodiffusion with class and subclass specific antisera (Litton Bionetics, Kensington, Md, USA).

The monoclonal antibody designated NP-2 was found to bind 60 to 70% of CEA and meconium antigen (MA)

(isolated by the method of Primus et al Cancer Res. (1983) 43, 679-685 and labelled by the chloramine-T method) but not nonspecific cross-reacting antigen (NCA). The affinity constant of NP-2 against CEA (the concentration of unlabelled CEA required to inhibit by 50% antibody binding to labelled CEA) was $6.6 \times 10^{-12}$M or 7.8ng/ml ($K = 1.9 \times 10^{11}$ $M^{-1}$).

Further routine details, such as the charcteristics of suitable radioimmunoassays, may be obtained if needed from Primus et al Cancer Res. (1983) 43:686-692.

B) The antibodies prepared above are labelled with carborane groups according to the procedure of part B of Example 1.

C) Another monoclonal anti-CEA antibody, prepared as described in part A, but with a different epitope specificity than the anti-CEA antibody labelled with carborane groups, is conjugated to glucose oxidase as described in part E of Example 1.

D) A liquid serum sample is placed int the polystyrene tube prepared according to part D of Example 1. Carborane-labelled anti-CSAp and anti-CEA monoclonal antibodies are then added to the tube along with peroxidase-labelled anti-CSAp and glucose oxidase-labelled anti-CEA monoclonal antibodies. The tube is incubated for 2 hours at 37°C and then washed with saline solution. The CSAp content of the sample is determined using buffered o-phenylenediamine and hydrogen perodixe, as described in part F of Example 1, and the tube is again washed once with normal saline. A solution consisting of 1.67% glucose, 0.005% o-dianisidine and 0.0007% peroxidase in 0.05M sodium acetate buffer, pH 5.1, is added and incubated for an additional 30 minutes at 22$^{o}$C to develop the glucose oxidase raction. The tube is then introduced into a spectrophotometer and readings are taken at 490nm. The glucose oxidase activity is determined, and correlated with CEA content of the sample by the use of a standard curve, determined with a series of dilutions of known concentrations of purified CEA.

## EXAMPLE 3

### Simultaneous Immunoassays for CSAp and CEA using Radiolabelled Probe Antibodies

A simultaneous assay is carried out as described in Example 2, except that instead of enzyme-labelled antibodies, I-125-labelled anti-CSAp and I-131-labelled anti-CEA monoclonal probe antibodies are used. CSAp and CEA quantities are correlated by differential measurement of the I-125 and I-131 radioactive levels in a two channel gamma scintillation counter.

## EXAMPLE 4

### Immunoassay Kit for Simultaneous Determinations of CEA

<u>and CSAp</u>

A typical kit for effecting the simultaneous assay described in Example 2 contains the following components:

-polystyrene tubes coated with anti-carborane antibodies (dry). It will be understood that coated microtiter plates can be substituted for the tubes, or that lyophilized anti-carborane antibodies can be supplied for custom applications, e.g., coating of automated assay media.

-lyophilized carborane-labelled anti-CEA and anti-CSAp antibodies.

-lyophilized glucose-oxidase-labelled amto-CEA and peroxidase-labelled anti-CSAp antibodies.

-lyophilized CEA and CSAp reference standards.

-peroxidase developing system, e.g., in pellet form, containing o-phenylenediamine and buffer salts.

-glucose oxidase developing system, e.g., in pellet form, containing peroxidase, O-dianisidine, glucose and buffer salts.

The user will normally provide distilled water, hydrogen peroxide and saline washing solution.

It will be understood that other configurations will be readily apparent to the skilled art worker, depending on the particular assay methods and instruments used. The foregoing kit is merely illustrative of a preferred embodiment.

The preceding examples can be repeated with similar success by substituting the generically or specifically described reactants and/or operating conditions of this invention for those used in the preceding examples. This includes the enzyme systems, hapten labels, radiolabels, fluorescent labels, solid supports, antibodies and the like described in the sources incorporated herein by reference as well as other conventional techniques and reagents.

From the foregoing description, one skilled in the art can easily ascertain the essential characteristics of this invention and, without departing form the spirit and scope thereof, can make various changes and modifications of the invention to adapt it to various usages and conditions.

## CLAIMS

1. An immunoassay method for determining the amount of a polyvalent ligand in a liquid test sample, the ligand being a binding substance or a substance capable of specific binding to a binding substance, the method comprising the steps of:

(a) providing an insoluble phase to which is bound a separation specific binding substance which does not bind the ligand;

(b) incubating the insoluble phase with

(1) the liquid test sample;

(2) a capture specific binding substance which specifically binds the ligand, and which is itself specifically bound by the separation specific binding substance; and

(3) a probe specific binding substance which specifically binds the ligand at a site which is accessible when the ligand is bound to the capture specific binding substance, the probe specific binding substance being not substantially bound by the separation specific binding substance, and being detectable by at least one detection procedure;

(c) separating the insoluble phase, after the incubation, from resultant liquid phase containing unbound reagents and components of the test sample; and

(d) determining, by means of the detection procedure, the amount of the detectable probe which is bound to the separated insoluble phase, or the amount of the detectable probe remaining in the resultant liquid phase, the determination being related to the amount of the ligand in the test sample.

2. An immunoassay method for determining the amount of each of a plurality of polyvalent ligands in a liquid test sample, each of the ligands being a binding substance or a substance capable of specific binding to a binding substance, the method comprising the steps of;

(a) providing an insoluble phase to which is bound at least one separation specific binding substance which does not bind any of the ligands;

(b) incubating the insoluble phase with

(1) the liquid test sample;

(2) for each of the ligands a capture specific binding substance which specifically binds that ligand, and which is itself specifically bound by the separation specific binding substance; and

(3) for each of the ligands, a probe specific binding substance which specifically binds that ligand at a site which is accessible when the ligand is bound to the capture specific binding substance, each of the probe specific binding substances being not substantially bound by the separation specific binding substance, and each being separately detectble by at least one detection procedure;

(c) separating the insoluble phase, after the incubation, from the resultant liquid phase containing unbound reagens and components of the test sample; and

(d) determining, by means of the detection procedure, the amount of each of the detectable probes which is bound to the separated insoluble phase, or the amount of the detectable probe remaining in the resultant liquid phase, the determination being related to the amount of the ligand in the test sample.

3. A method as claimed in Claim 1 or 2, wherein the ligand (or each of them) is an antibody, and the capture specific binding substance (or each of them) comprises a specific binding complement to the antibody.

4. A method as claimed in Claim 1, 2 or 3, wherein each of the probe specific binding substance and the capture specific binding substance (or each of them) comprises an antibody, and the ligand (or each of them) is a specific binding complement to both of the antibodies.

5. A method as claimed in Claim 1, wherein the capture specific binding substance (or each of them) is conjugated to a hapten, and the separation specific binding substance is an antibody which specifically binds the hapten.

6. An immunoassay kit, suitable for determining the amount of a polyvalent ligand in a liquid test sample, the kit comprising:

(a) an insoluble phase to which is bound a separation specific binding substance which does not bind the ligand;

(b) a capture specific binding substance which specifically binds the ligand, and which is itself capable of being specifically bound by the separation specific binding substance; and

(c) a probe specific binding substance which specifically binds the ligand at a site which is accessible when the ligand is bound to the capture specific binding substance, the probe specific binding substance being not substantially bound by the separation specific binding substance, and being detectable by at least one detection procedure.

7. An immunoassay kit, suitable for determining the amount of each of a plurality of polyvalent ligands in a liquid test sample, each of the ligands being a binding substance or a substance capable of specific binding to a binding substance, the kit comprising;

(a) an insoluble phase to which is bound at least one separation specific binding substance which does not bind any of the ligands;

(b) for each ligand, a capture specific binding substance which specifically binds that ligand, and which is itself specifically bound by the separation specific binding substance; and

(c) for each ligand, a probe specific binding substance which specifically binds that ligand at a site which is accessible when the ligand is bound to the capture specific binding substance, each probe specific binding substance being not substantially bound by the separation specific binding substance, and each being separately detectable by at least one detection procedure.

8. A kit as claimed in Claim 6 or 7, the kit being suitable for use wherein the ligand (or each of them) is an antibody and wherein the capture specific binding substance (or each of them) comprises an antigen which is a specific binding complement to the antibody.

9. A kit as claimed in Claim 7, suitable for the simultaneous determination of CEA and CSAp, wherein one of the capture specific binding substances and one of the probe specific binding substances is a monoclonal antibody which specifically binds CEA, and one of the capture specific binding substances and one of the probe specific binding substances is a monoclonal antibody which specifically binds CSAp.

10. A kit as claimed in Claim 7, wherein each of the capture antibodies is conjugated to a carborane moiety; and wherein the separation specific binding substance comprises an antibody which specifically binds the carborane moiety.